# EUROPEAN PATENT SPECIFICATION

(11) **EP 3 766 459 B1**
(45) Date of publication and mention of the grant of the patent: **28.08.2024**
(21) Application number: 20185848.7
(22) Date of filing: 15.07.2020
(51) Int. Cl.: A61F 2/44, A61F 2/46, A61B 17/88

(54) **LATERALLY INSERTABLE INTERVERTEBRAL SPINAL IMPLANT**
SEITLICH EINSETZBARES ZWISCHENWIRBELSÄULENIMPLANTAT
IMPLANT VERTÉBRAL INTERVERTÉBRAL INSÉRABLE LATÉRALEMENT

(30) Priority: 18.07.2019 US 201916515780
(43) Date of publication of application: 20.01.2021
(73) Proprietor: Globus Medical, Inc., Audubon, PA 19403 (US)
(72) Inventor: BURKHARDT, Alex, AKRON, PA 17501 (US); ISRAEL, Jenna, PHILADELPHIA, PA 19129 (US); FRIEDRICH, Adam, CINNAMINSON, NJ 08077 (US)
(74) Representative: Morabito, Sara

(56) References cited:
- US-A1- 2006 085 071
- US-A1- 2012 290 089
- US-A1- 2014 277 497
- US-A1- 2015 190 241
- US-A1- 2015 374 510

## Description

### FIELD

The present disclosure generally relates to fixation devices and systems for positioning and immobilizing at least two adjacent vertebrae and methods (not claimed) related to the same In particular, the present disclosure relates to interbody fusion devices with angled fixation holes configured to facilitate lateral arterial implantation.

### BACKGROUND

The spine is the axis of the skeleton on which all of the body parts "hang". In humans, the normal spine has seven cervical, twelve thoracic and five lumbar segments. The lumbar spine situs upon the sacrum, which then attaches to the pelvis, and in turn is supported by the hip and leg bones. The bony vertebral bodies of the spine are separated by intervertebral discs, which act as joints but allow known degrees of flexion, extension, lateral bending, and axial rotation.

The typical vertebra has a thick anterior bone mass called the vertebral body, with a neural (vertebral) arch that arises from the posterior surface of the vertebral body. The central of adjacent vertebrae are supported by intervertebral discs. The spinal disc and/or vertebral bodies may be displaced or damaged due to trauma, disease, degenerative defects, or wear over an extended period of time. One result of this displacement or damage to a spinal disc or vertebral body may be chronic back pain. In many cases, to alleviate back pain from degenerated of herniated discs, the disc is removed along with all or part of at least one neighboring vertebrae and is replaced by an implant that promotes fusion of the remaining bony anatomy.

Although most spinal surgeries are performed using a posterior (back) approach, in some cases a surgeon may choose an anterior (ALIF) approach for various reasons, for example, to allow more direct access to the intervertebral disk; to have the ability to add more lordosis (swayback) to the d spine; and to provide access to the spine without moving the nerves. Treatment of the disc at the L5/S1 level is particularly suitable for the ALIF approach due to the efficient vascular access below with bifurcation of the aorta and inferior vena cava. However, the ALIF approach typically requires organs and blood vessels be moved to the side. As such, in many cases, a vascular surgeon assists the orthopaedic surgeon with opening and exposing the disk space.

Additionally, the ALIF approach is typically performed with the patient in a supine position. As such, other procedures, for example, attaching a plate or rod to posterior spine, will generally require changing the position of the patient to provide posterior axis. The result is often increased surgical time and reduced surgical workflow. Alternatively, US 2014/277497 A1 and US 2015/190241 A1 disclose surgical systems employed with a method including an oblique lateral interbody fusion (OLIF) procedure.

### SUMMARY

To meet this and other needs, an intervertebral implant has an overall footprint that matches that of a standard integrated-fixation ALIF, however, the means of attachment to the device and the angle at which the fixation is delivered, sit at an angle relative to the disc space.

The present invention relates to an intervertebral implant set forth in claim 1 and a kit with a corresponding insertion tool set forth in claim 8. Preferred embodiments of the invention are set forth in the dependent claims.

According to at least one embodiment of the disclosure, an insertion tool and intervertebral implant kit is disclosed. The implant includes a body having a front end, a rear end and a pair of spaced apart first and second side walls extending between the front and rear ends. The front and rear ends extend in a transverse direction and a central axis of the body extends from the rear end to the front end. The rear end defines a first fastener hole having a first central axis and a second fastener hole having a second central axis. The first and second central axes extend parallel to one another at an acute angle relative to the body central axis in the transverse direction. The insertion tool includes a tool body extending from a proximal end to distal end. The distal end defines a face and the tool body defines at least two fastener passages with respective third and fourth central axes. The insertion tool is configured to support the implant such that the implant rear end extends along the face and the third and fourth central axes align with and are parallel to the first and second central axes, respectively, in the transverse direction.

According to the disclosure, a retraction assembly (not claimed) is disclosed. The retraction assembly includes a mounting plate with at least one mount extending therefrom. The mounting plate has a chamber extending therein with an adjustment screw extending into the chamber. A lateral adjustment arm has a first end with a shaft which is positioned in the chamber and engages the adjustment screw such that rotation thereof causes the lateral adjustment arm to move laterally relative to the mounting plate. The second end of the lateral adjustment arm defines a pivot mount. A pivot member is pivotally connected to the lateral adjustment arm at the pivot mount with a second adjustment screw extending from the lateral adjustment arm and engaging the pivot member such that rotation thereof causes pivoting of the pivot member relative to the lateral adjustment arm. A retraction blade connected to the pivot member.

### BRIEF DESCRIPTION OF THE DRAWINGS

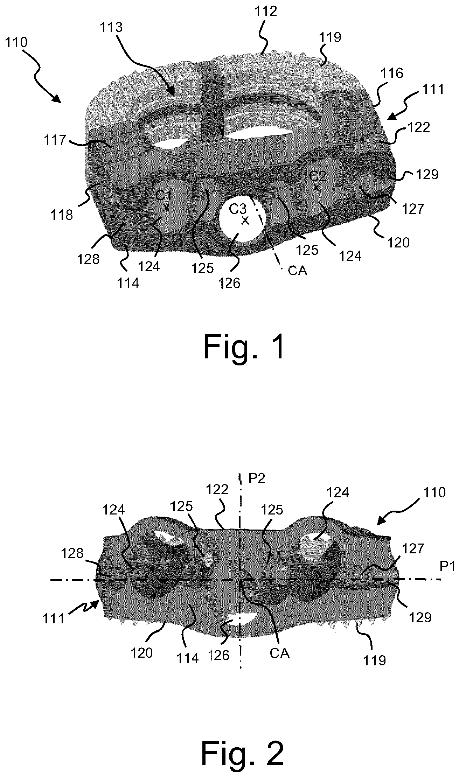

The embodiments of the invention are illustrated in fig. 1 to 7. The examples shown in the other figures and described in paragraphs [0025] to [0030] do not form part of the invention but represent background art that is useful for understanding the invention. A more complete understanding of the present disclosure, and the attendant advantages and features thereof, will be more readily understood by reference to the following detailed description when considered in conjunction with the accompanying drawings wherein:
Fig. 1 is a bottom perspective view of an intervertebral implant according to an embodiment of the disclosure.
Fig. 2 is anterior elevation view of the intervertebral implant of Fig. 1.
Fig. 3 is bottom plan view of the intervertebral implant of Fig. 1.
Fig. 4 is a perspective view similar to Fig. 1 illustrating fasteners positioned within the implant.
Fig. 5 is a cross-sectional view of the anterior portion of the implant of Fig. 1 with a locking screw positioned therein.
Fig. 6 is a top plan view of the intervertebral implant of Fig. 1 positioned relative to a vertebra.
Fig. 7 is a perspective view illustrating the intervertebral implant of Fig. 1 connected to an insertion tool in accordance with an embodiment of the disclosure.
Figs. 8-10 are perspective, side and elevation views, respectively, of a retraction blade in accordance with an example of the disclosure.
Figs. 11-13 are perspective, side and elevation views, respectively, of a retraction blade in accordance with another an example of the disclosure.
Fig. 14 is a perspective view of the retraction blade of Figs. 8-10 positioned in a mounting system in accordance with an example of the disclosure.

### DETAILED DESCRIPTION

Embodiments of the disclosure are generally directed to intervertebral implants, systems, and method of use thereof (not claimed).

Referring to Figs. 1-7, an embodiment of a lateral anterior lumbar interbody fusion implant 110 in accordance with an embodiment of the disclosure will be described. As illustrated, the implant 110 has a body 111 with a generally D-shaped configuration. The body 111 is defined by a posterior end 112, an anterior end 114 and side walls 116 and 118 extending therebetween. A hollow interior chamber 113 is defined within an inner perimeter of the body 111. The hollow interior chamber 113 is configured to receive bone growth promoting materials. The implant 110 has an upper surface 120 and a substantially parallel lower surface 122. It is noted that in Figs. 1-4, the implant 110 is illustrated in an inverted position such that the lower or superior surface 122 is facing upward. The upper and lower surfaces 120, 122 define a plurality of serrations 117 along the side walls 116, 118 and a plurality of serrations 119 along the posterior end 112.

While the implant 110 is illustrated and described with a body 111 having a specific configuration, the disclosure is not limited to such. The body 111 may have various other configurations suitable for the disc space into which the implant 110 is intended. For example, the posterior end 112 may be formed with a taper or the implant 110 may have a wedge shape such that the entire body 111 tapers from the anterior end 114 to the posterior end 112. Similarly, the side walls 116, 118 may be angled toward one another rather than extending substantially parallel to one another. As yet another example, the body 111 may have an adjustable configuration. In each case, the implant body 111 will have a central axis CA extending from the anterior end 114 to the superior end 112. The central axis CA is located at the junction between the mid transverse plane P1 and the mid sagittal plane P2 and extends in each of the planes P1, P2 (see Fig. 2).

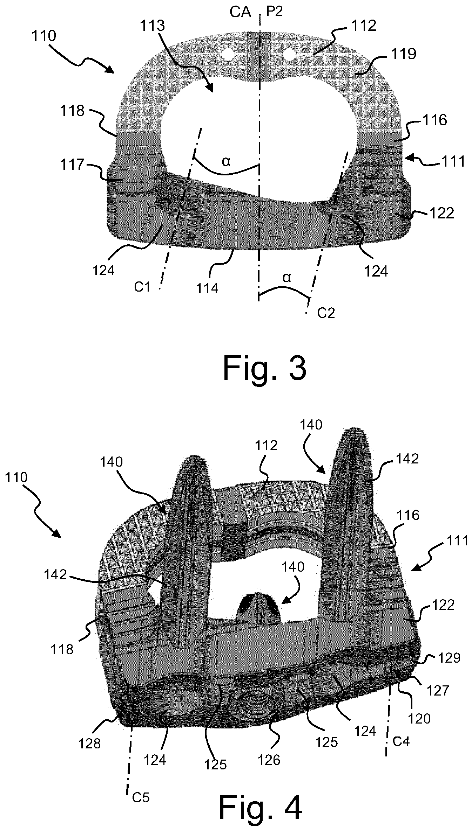

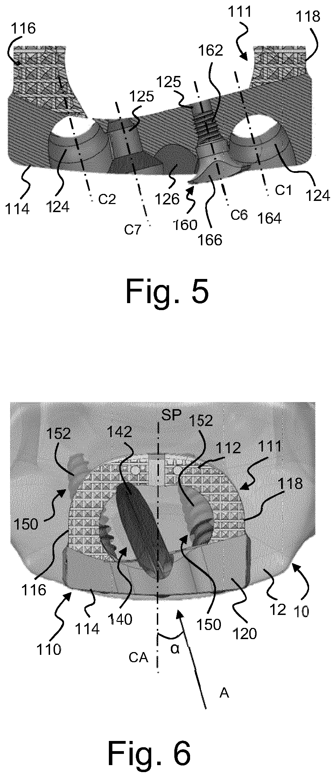

The anterior end 114 of the implant 110 includes a plurality of fastener holes 124, 126 through which anchors 140 or screws 150 (see Figs. 4 and 6) extend to anchor the implant onto the vertebral body. Each of the fastener holes 124, 126 has a central axis C1, C2, C3. As is known in the art, the axes C1, C2, C3 may be angled superiorly or inferiorly relative to the transverse plane P1 such that the fasteners will be directed toward the superior or inferior vertebral body. In the illustrated embodiment, the axes C1, C2 of fastener holes 124 each extend inferiorly relative to the transverse plane P1 and the axis C3 of the fastener hole 126 extends superiorly relative to the transverse plane P1. Such configuration is not required and the implant may have other configurations with more or fewer holes and with different numbers of holes extending superiorly or inferiorly. Additionally, when utilizing anchors 140 such angling may not be as necessary or necessary at all since the curved configuration of the anchor blade body 142 is curved and will naturally extend superiorly or inferiorly.

Secondary holes 125 are provided to receive respective blocking set screws 160 (see Fig. 5). The secondary holes 125 each extend along a central axis C6, C7. Each of the secondary hole axes C6, C7 is generally parallel to the transverse plane P1. A blind hole 127 with a slot 129 and a threaded hole 128 are provided for receiving an instrument 170 that is used for inserting the implant 110, as will be described hereinafter. The central axis C4 of the blind hole 127 and the central axis C5 of the threaded hole 128 also are generally parallel to the transverse plane P1.

In addition to the angular orientation in the superior/inferior direction, each of the axes C1-C7 is also angled relative to the sagittal plane P2 (and thereby the central axis CA) in the transverse direction to facilitate lateral insertion of the implant 110. Referring to Fig. 3, the axes C1 and C2 are parallel to one another and each are angled relative the sagittal plane P2 at an acute angle α. While not shown in Fig. 3, the axis C3 of fastener hole 126 will also be angled relative the sagittal plane P2 at the acute angle α and will be parallel to the axes C1 and C2. As seen in Fig. 6, upon fixation of the implant 110 relative to the vertebral body 12 of the spine 10, the shaft 142, 152 of each of the fasteners 140, 150 will parallel to each other in the transverse direction, each extending at the angle α relative to the central axis CA of the implant 110.

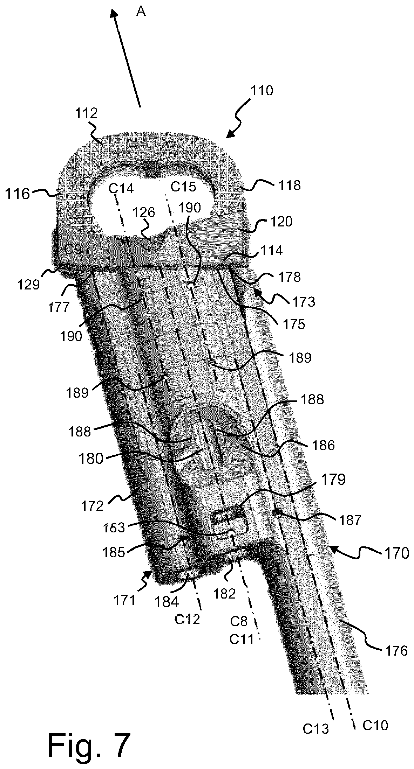

The angle α is chosen to approximate the angle of the lateral insertion path A of the implant relative to the sagittal plane SP of the vertebral body 12 of the spine 10 as shown in Fig. 6. As shown in Fig. 7, such allows the insertion tool 170 to hold the implant for insertion along the path A and also allows the fasteners 140, 150 to be delivered along the same path along which the insertion tool will extend. Upon proper positioning, the central axis CA of the implant 110 will extend approximately along the sagittal plane SP of the vertebral body 12 and the fasteners 140, 150 may be easily directed into engagement with the superior and inferior vertebral bodies.

Referring to Fig. 4, to facilitate easy mounting of the insertion tool 170 relative to implant 110, the axis C4 of the blind hole 127 and the axis C5 of the threaded hole 128 also extend parallel to the fastener hole axes C1-C3. As such, the insertion tool 170 may be delivered to the implant 110 along the path A and then a threaded connector (not shown) can be advanced parallel to the axis of the tool 170.

Referring to Fig. 5, to facilitate easy engagement of the blocking set screws 160, the axes C6, C7 of the secondary holes 125 also preferably extend parallel to the fastener hole axes C1-C3. With the shaft 162 of each blocking set screw 160 extending into a respective secondary hole 125, the head 164 thereof will be co-axial with the secondary hole 125. As such, a driver (not shown) may be moved along at the same angle defined by the insertion tool 170 to engage the head 164 and move the set screw 160 from a non-blocking position, wherein the recess(es) 166 on the head 164 are aligned with a respective fastener hole 124, 126, to a blocking position wherein the head 164 overlies the fastener holes 124, 126.

Having generally described the implant 110, the insertion tool 170 and implant procedure will be described in more detail with reference to Fig. 7. The insertion tool 170 includes a main body 172 extending from a proximal end 171 to a distal end 173 along a tool central axis C8. The distal end 173 defines a face 175 configured to engage the anterior end 114 of the implant 110. The face 175 is angled relative to the tool central axis C8 such that upon mounting of the implant 110 on the tool 170, the tool central axis C8 will be parallel with the central axes C1-C7 of the implant in the transverse direction. The tool body 172 may define one or more openings 179 configured to mount other tools thereto, for example, an articulating table arm or a pushing handle (not shown).

To connect the insertion tool 170 to the implant 110, a pin (not shown) extends from the tool face 175 at the location labeled 177 which aligns with the blind hole 127 of the implant. The pin extends along an axis C9 which is parallel to the tool central axis C8 such that the pin will extend into the blind hole 127 and the face 175 will extend along the anterior end 114 of the implant 110. A threaded connector (not shown) extends through a passage in the insertion tool 170 such that the threaded connector extends from the face 175 at the location labeled 178 which aligns with the threaded hole 128. The tool 170 may include a handle portion 176 through which the connector passage extends. The threaded connector passage extends along an axis C10 that is parallel with the tool central axis C8 such that the threaded connector can be advanced into and threadably engage the threaded hole 128, thereby mounting the implant 110 to the face 175 of the insertion tool 170.

The tool body 172 defines fastener passages 182, 184 (only two shown in Fig. 7) configured to align with each of the fastener holes 124, 126. The axes C11, C12, C13 of the fastener passages 182, 184 extend parallel to the tool central axis C8. As such, the fasteners 140, 150 may be easily passed through the insertion tool 170 into the respective fastener hole 124, 126. In the event a curved anchor blade 142 is utilized, the passages 182, 184 may have corresponding curves (in the superior/inferior direction). An alignment hole 183, 185, 187 extends into each of the fastener passages 182, 184.

To access the blocking set screws 160, an opening 186 extends into the body 172 to set screw passages 188 on either side of the tube 180 defining the fastener passage 182. Each set screw passage 188 aligns with a respective secondary hole 125. The axes C14, C15 of the set screw passages 188 extend parallel to the tool central axis C8. As such, a drive tool (not shown) may be passed through each set screw passage 188 to engage and rotate a respective set screw 160. Alignment holes 189, 190 extend into each of the set screw passages 188.

While the illustrated embodiments have a fixed angle α for the holes and tool passages, it is possible to make the angle adjustable such that the implant 110 may be adjustable for different anatomies. For example, each of the implant holes could include a ball and socket configuration which is lockable at a desired angle. The face of the insertion tool could be pivotably adjustable to match the angle set for the implant holes. Other means for adjusting the angle of the holes and the tool passages may also be utilized.

The implant 110 and insertion tool 170 provide greater ease of use off-axis to disc spaces, for example, the L5-S1 disc space. Traditional ALIF implants require a straight-on approach, which is made more difficult when the patient is positioned on their side. The angled approach to the disc space with the angled tool, paired with a matching angle by which the fixation is delivered and blocked in place facilitates operating on the L5-S1 disc space, or other desired disc spaces, via a lateral position, or "lateral ALIF".

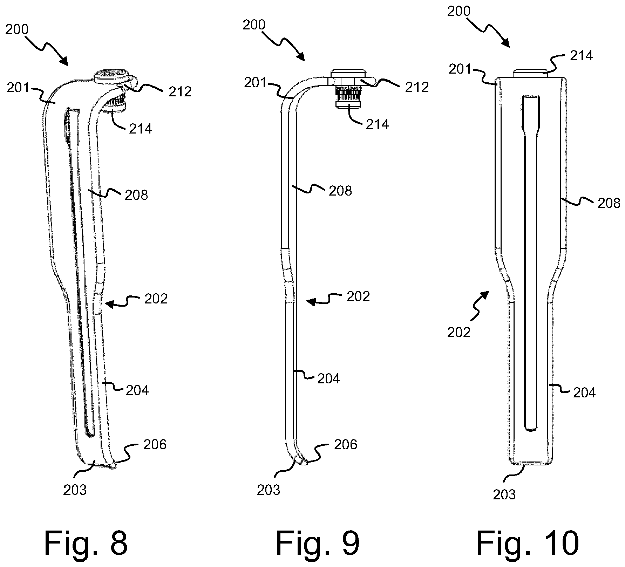

Such lateral ALIF requires retraction of different anatomy to access the disc space with a patient on their side. Referring to Figs. 8-14, a retraction assembly 220 which offers increased ability to adjust blade position as well as specific options for retracting the anatomy as it pertains to accessing the disc space with a patient on their side will be described.

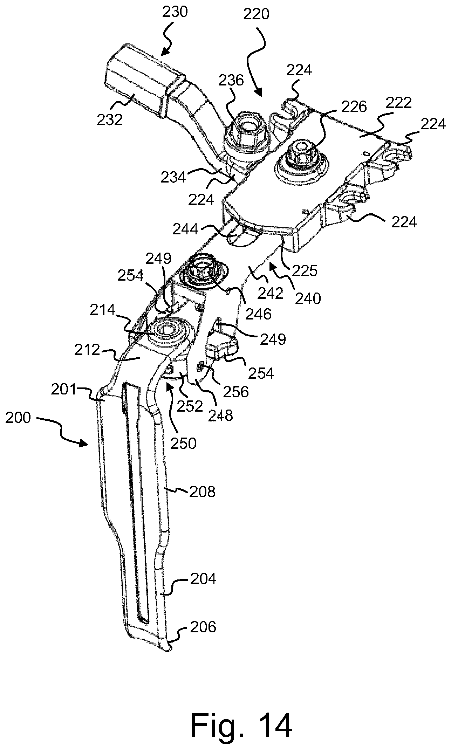

Referring to Figs. 8-10, an illustrative blade 200 for use with the retraction assembly 220 illustrated in Fig. 14 will be described. The blade 200 includes a body 202 extending from a proximal end 201 to a distal end 203. The distal end 203 of the body 202 includes a portion 204 having a narrower width than the portion 208 at the proximal end 201. The body 202 may have a lateral curvature as illustrated. Additionally, a curved tip 206 is defined at the distal end 203 of the body 202. The curved tip 206 may also narrow as it moves distally. The narrow portion 204 and the curved tip 206 allow for retraction of vasculature, for example, at the crotch of the bifurcation. The wider portion 208 at the proximal end 201 provides better retraction of soft tissue. The bi-functionality of the blade 200 serves well for a minimally invasive anterior approach where the vessels need to be mobilized. A flange portion 212 extends from the proximal end 201 and supports a connecting member 214 configured to engage and mount to a pivoting member 250 of the retraction assembly 220 as will be described hereinafter.

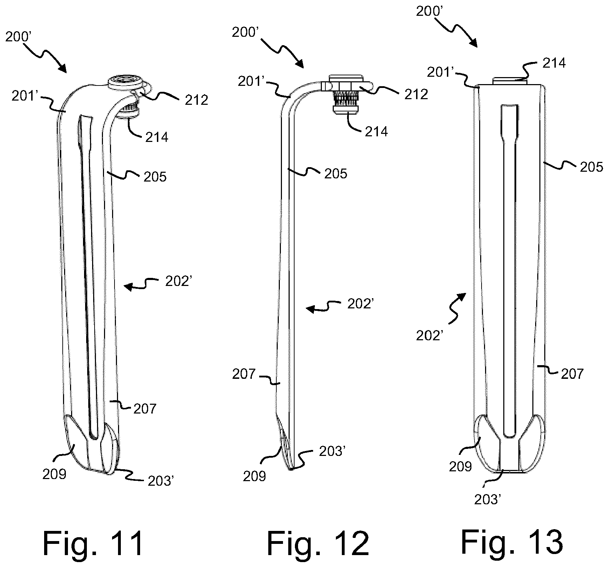

Referring to Figs. 11-13, another illustrative blade 200' for use with the retraction assembly 220 illustrated in Fig. 14 will be described. The blade 200' includes a body 202' extending from a proximal end 201' to a distal end 203'. The body 202' tapers in thickness from a thinner portion 205 at the proximal end 201' to a thicker portion at the distal end 203'. This configuration serves to stiffen the blade 200'. Again, the body 202' may have a lateral curvature as illustrated. Additionally, a scalloped tip 209 is defined at the distal end 203' of the body 202'. The scalloped tip 209 may be used to sit under bony anatomy and act as a lever for retracting soft tissue. Again, a flange portion 212 extends from the proximal end 201' and supports a connecting member 214 configured to engage and mount to a pivoting member 250 of the retraction assembly 220.

Referring to Fig. 14, a retraction assembly 220 in accordance with an example of the disclosure will be described. The retraction assembly 220 includes a mounting plate 222 with one or more mounts 224 extending therefrom. The mounts 224 are configured for mounting directly to an articulating table arm 230 or other structure, for example, mounting to extensions which mount to a more traditional ring-based design. In the illustrated example, the table arm 230 includes an arm 232 which terminates in a screw platform 234 supporting a connecting screw 236. The connecting screw 236 is positioned within the mount 224 and tightened such that the mount 234 is secured between the head of the screw236 and the screw platform 234 to secure the mounting plate 222.

A cavity 225 extends into the mounting plate 222 and is configured to receive a shaft 242 of a lateral adjustment arm 240. The shaft 242 defines a slot 244 into which an adjustment screw 226 of the mounting plate 222 extends. The screw 226 engages within the slot 244 and thereby defines the range of later movement of the lateral adjustment arm 240. The screw 226 and slot 244 may have various adjustment configurations, for example, a friction lock, gear assembly, or a rack and pinion arrangement.

The opposite end of the lateral adjustment arm 240 defines a fork 248 with a pair of openings 249 on each side to support a pivot member 250. The pivot member 250 includes a body 252 with an opening configured to receive the blade connecting member 214 to mount the blade 200 on the assembly 220. A pivot pin 256 extends through the fork 248 and end of the pivot member body 250 such that the pivot member 250 is pivotally supported relative to the fork 248. A pair of opposed extensions 254 extend into the openings 249 and define the range of pivot. An adjustment screw 246 on the lateral adjustment arm 240 engages an opposite end of the pivot member 250 such that rotation thereof causes the pivot member 250, and thereby the blade 200, to pivot. Pivoting of the blade 200 allows the blade 200 to change angulation to compensate for various anatomy and tissue.

Although the invention has been described in detail and with reference to specific embodiments, it will be apparent to one skilled in the art that various changes and modifications can be made without departing from the scope of the invention. Thus, it is intended that the invention covers the modifications and variations of this invention provided they come within the scope of the appended claims. It is expressly intended, for example, that all ranges broadly recited in this document include within their scope all narrower ranges which fall within the broader ranges. It is also intended that the components of the various devices disclosed above may be combined or modified in any suitable configuration.

## Claims

1. An intervertebral implant (110) for implantation in an intervertebral space between vertebrae, the implant comprising:
- a body (111) having a front end (112), a rear end (114) and a pair of spaced apart first and second side walls (116, 118) extending between the front (112) and rear (114) ends with the front (112) and rear (114) ends extending in a transverse direction and a central axis (CA) of the body (111) extending from the rear end (114) to the front end (112),
- wherein the rear end (114) defines a first fastener hole (124) having a first central axis (C1) and a second fastener hole (126) having a second central axis (C2), the first (C1) and second (C2) central axes lying on respective planes extending parallel to one another and at an acute angle relative to a sagittal plane (P2) of the body central axis (CA) in the transverse direction,
- wherein the rear end (114) includes a threaded insertion tool receiving hole (128) on a portion adjacent to the second side wall (118) and the rear end (114) further includes a blind hole (127) with a slot (129) on a portion adjacent the first side wall (116), wherein the threaded insertion tool receiving hole (128), the blind hole (127), and the slot (129) are configured to receive an insertion tool (170).

2. The intervertebral implant of claim 1, wherein the rear end (114) defines a third fastener hole (124) having a third central axis (C3) lying on a plane which is parallel to the first (C1) and second axes (C2) in the transverse direction.

3. The intervertebral implant of claim 1, wherein the first central axis (C1) extends in a first direction relative to a superior/inferior direction and the second central axis (C2) extends in a second opposite direction relative to the superior/inferior direction.

4. The intervertebral implant of claim 1, wherein at least one blocking set screw hole (125) is defined by the rear end (114) adjacent at least one of the fastener holes (124), the blocking set screw hole (125) having a fourth central axis (C4) lying on a plane which is parallel to the first (C1) and second (C2) axes in the transverse direction.

5. The intervertebral implant of claim 4, further comprising a blocking set screw (160) positioned within the blocking set screw hole (125) and moveable between a non-blocking position and a blocking position.

6. The intervertebral implant of claim 1, wherein the insertion tool receiving hole (128) has a fifth central axis (C5) lying on a plane which is parallel to the first (C1) and second (C2) axes in the transverse direction.

7. The intervertebral implant of claim 1, further comprising a fastener (140, 150) positioned in each fastener hole (124, 126), each of the fasteners (140, 150) including a shaft (142, 152) and the shafts (142, 152) lie on planes that are parallel to one another in the transverse direction.

8. An insertion tool (170) and intervertebral implant (110) kit, the kit comprising:
- an implant comprising: an implant body (111) having a front end (112), a rear end (114) and a pair of spaced apart first and second side walls (116, 118) extending between the front (112) and rear (114) ends with the front (112) and rear (114) ends extending in a transverse direction and a central axis (CA) of the implant body (111) extending from the rear end (114) to the front end (112) , wherein the rear end (114) defines a first fastener hole (124) having a first central axis (C1) and a second fastener hole (126) having a second central axis (C2), the first (C1) and second (C2) central axes having planes extending parallel to one another at an acute angle relative to a plane of the implant body (111) central axis (CA) in the transverse direction, wherein the rear end (114) includes a threaded insertion tool receiving hole (128) on a portion adjacent to the first side wall (116) and the rear end (114) further includes a blind hole (127) with a slot (129) on a portion adjacent the second side wall (118), wherein the threaded insertion tool receiving hole (128), the blind hole (127), and the slot (129) are configured to receive an insertion tool (170); and
- an insertion tool (170) comprising: a tool body (172) extending from a proximal end (171) to distal end (173), the distal end (173) defining a face (175) and the tool body (172) defining at least two fastener passages with respective third (C4) and fourth (C5) central axes,
- wherein the insertion tool (170) is configured to support the implant (110) such that the implant rear end (114) extends along the face (175) and planes of the third (C4) and fourth (C5) central axes align with and are parallel to planes of the first (C1) and second (C2) central axes, respectively, in the transverse direction.

9. The kit of claim 8, wherein the implant rear end (114) defines a third fastener hole (124) having a fifth central axis (C3) with a plane which is parallel to planes of the first (C1) and second (C2) axes in the transverse direction and the insertion tool body (172) defines a third fastener passage having a sixth central axis having a plane which aligns with and is parallel to the plane of the fifth central axis (C3) in the transverse direction when the implant (110) is supported on the insertion tool.

10. The kit of claim 8, wherein the first central axis (C1) extends in a first direction relative to a superior/inferior direction and the second central axis (C2) extends in a second opposite direction relative to the superior/inferior direction.

11. The kit of claim 8, wherein at least one blocking set screw hole (125) is defined by the implant rear end (114) adjacent at least one of the fastener holes (124), the blocking set screw hole (125) having a seventh central axis having a plane which is parallel to the planes of the first (C1) and second (C2) axes in the transverse direction and the insertion tool body (172) defines a driver passage having an eighth central axis having a plane which aligns with and is parallel to the plane of the seventh central axis in the transverse direction when the implant (110) is supported on the insertion tool (170).

12. The kit claim 11, further comprising a blocking set screw (160) positioned within the blocking set screw hole (125) and moveable between a non-blocking position and a blocking position by a driver positioned within the driver passage.

13. The kit of claim 8, wherein at least one insertion tool receiving hole (125) is defined by the rear end (114) and has a ninth central axis having a plane which is parallel to the planes of the first (C1) and second (C2) axes in the transverse direction and a mounting element extends from the face (172) of the insertion tool body (170) and is configured to be received in the insertion tool receiving hole (125).

14. The kit of claim 8, further comprising a fastener (140, 150) positioned in each fastener hole (124, 126), each of the fasteners (140, 150) including a shaft (142, 152) and the shafts (142, 152) lie on planes that are parallel to one another in the transverse direction.

## Patentansprüche

1. Zwischenwirbelimplantat (110) zur Implantation in einen Zwischenwirbelraum zwischen Wirbeln, wobei das Implantat aufweist:
- einen Körper (111) mit einem vorderen Ende (112), einem hinteren Ende (114) und einem Paar von beabstandeten ersten und zweiten Seitenwänden (116, 118), die sich zwischen dem vorderen (112) und hinteren (114) Ende erstrecken, wobei sich das vordere (112) und hintere (114) Ende in eine Querrichtung erstrecken und sich eine Mittelachse (CA) des Körpers (111) vom hinteren Ende (114) zum vorderen Ende (112) erstreckt,
- wobei das hintere Ende (114) ein erstes Befestigungsloch (124) mit einer ersten Mittelachse (C1) und ein zweites Befestigungsloch (126) mit einer zweiten Mittelachse (C2) bildet, wobei die erste (C1) und zweite (C2) Mittelachse auf jeweiligen Ebenen liegen, die sich parallel zueinander und in einem spitzen Winkel relativ zu einer Sagittalebene (P2) der Körpermittelachse (CA) in Querrichtung erstrecken,
- wobei das hintere Ende (114) ein mit einem Gewinde versehenesEinsetzwerkzeug-Aufnahmeloch (128) an einem Abschnitt, angrenzend an die zweite Seitenwand (118), umfasst und das hintere Ende (114) ferner ein Sackloch (127) mit einem Schlitz (129) an einem Abschnitt, angrenzend an die erste Seitenwand (116), umfasst, wobei das mit dem Gewinde versehene Einsetzwerkzeug-Aufnahmeloch (128), das Sackloch (127) und der Schlitz (129) eingerichtet sind, ein Einsetzwerkzeug (170) aufzunehmen.

2. Zwischenwirbelimplantat nach Anspruch 1, wobei das hintere Ende (114) ein drittes Befestigungsloch (124) mit einer dritten Mittelachse (C3) bildet, die auf einer Ebene liegt, die parallel zur ersten (C1) und zweiten Achse (C2) in Querrichtung ist.

3. Zwischenwirbelimplantat nach Anspruch 1, wobei sich die erste Mittelachse (C1) in eine erste Richtung relativ zu einer oberen/unteren Richtung erstreckt und sich die zweite Mittelachse (C2) in eine zweite, entgegengesetzte Richtung relativ zur oberen/unteren Richtung erstreckt.

4. Zwischenwirbelimplantat nach Anspruch 1, wobei zumindest ein blockierendes Stellschraubenloch (125) durch das hintere Ende (114), angrenzend an zumindest eines der Befestigungslöcher (124), ausgebildet ist, wobei das blockierende Stellschraubenloch (125) eine vierte Mittelachse (C4) aufweist, die in einer Ebene liegt, die parallel zur ersten (C1) und zweiten (C2) Achse in Querrichtung ist.

5. Zwischenwirbelimplantat nach Anspruch 4, das ferner eine blockierende Stellschraube (160) aufweist, die im blockierenden Stellschraubenloch (125) positioniert ist und zwischen einer nicht blockierenden Position und einer blockierenden Position beweglich ist.

6. Zwischenwirbelimplantat nach Anspruch 1, wobei das Einsetzwerkzeug-Aufnahmeloch (128) eine fünfte Mittelachse (C5) aufweist, die in einer Ebene liegt, die parallel zur ersten (C1) und zweiten (C2) Achse in Querrichtung ist.

7. Zwischenwirbelimplantat nach Anspruch 1, das ferner ein Befestigungselement (140, 150) aufweist, das in jedem Befestigungsloch (124, 126) positioniert ist, wobei jedes der Befestigungselemente (140, 150) einen Schaft (142, 152) umfasst und die Schäfte (142, 152) in Ebenen liegen, die in Querrichtung parallel zueinander sind.

8. Kit mit Einsetzwerkzeug (170) und Zwischenwirbelimplantat (110), wobei das Kit aufweist:
- ein Implantat, umfassend: einen Implantatkörper (111) mit einem vorderen Ende (112), einem hinteren Ende (114) und einem Paar beabstandeter erster und zweiter Seitenwände (116, 118), die sich zwischen dem vorderen (112) und hinteren (114) Ende erstrecken, wobei sich das vordere (112) und hintere (114) Ende in eine Querrichtung erstrecken und sich eine Mittelachse (CA) des Implantatkörpers (111) vom hinteren Ende (114) zum vorderen Ende (112) erstreckt, wobei das hintere Ende (114) ein erstes Befestigungsloch (124) mit einer ersten Mittelachse (C1) und ein zweites Befestigungsloch (126) mit einer zweiten Mittelachse (C2) bildet, wobei die erste (C1) und zweite (C2) Mittelachse Ebenen aufweisen, die sich parallel zueinander in einem spitzen Winkel relativ zu einer Ebene der Mittelachse (CA) des Implantatkörpers (111) in Querrichtung erstrecken, wobei das hintere Ende (114) ein mit einem Gewinde versehenen Einsetzwerkzeug-Aufnahmeloch (128) an einem Abschnitt, angrenzend an die erste Seitenwand (116), umfasst und das hintere Ende (114) ferner ein Sackloch (127) mit einem Schlitz (129) an einem Abschnitt, angrenzend an die zweite Seitenwand (118), umfasst, wobei das mit einem Gewinde versehene Einsetzwerkzeug-Aufnahmeloch (128), das Sackloch (127) und der Schlitz (129) eingerichtet sind, ein Einsetzwerkzeug (170) aufzunehmen; und
- ein Einsetzwerkzeug (170), umfassend: einen Werkzeugkörper (172), der sich von einem proximalen Ende (171) zu einem distalen Ende (173) erstreckt, wobei das distale Ende (173) eine Fläche (175) bildet und der Werkzeugkörper (172) zumindest zwei Befestigungselementdurchgänge mit jeweiligen dritten (C4) und vierten (C5) Mittelachsen bildet,
- wobei das Einsetzwerkzeug (170) eingerichtet ist, das Implantat (110) abzustützen, so dass sich das hintere Ende (114) des Implantats entlang der Fläche (175) erstreckt und die Ebenen der dritten (C4) und vierten (C5) Mittelachse mit den Ebenen der ersten (C1) bzw. zweiten (C2) Mittelachse in Querrichtung ausgerichtet und parallel zu diesen sind.

9. Kit nach Anspruch 8, wobei das hintere Ende des Implantats (114) ein drittes Befestigungsloch (124) bildet, das eine fünfte Mittelachse (C3) mit einer Ebene aufweist, die parallel zu den Ebenen der ersten (C1) und zweiten (C2) Achse in Querrichtung ist und der Einsetzwerkzeugkörper (172) einen dritten Befestigungsdurchgang mit einer sechsten Mittelachse bildet, die eine Ebene aufweist, die mit der Ebene der fünften Mittelachse (C3) in Querrichtung ausgerichtet und parallel zu dieser ist, wenn das Implantat (110) auf dem Einsetzwerkzeug abgestützt wird.

10. Kit nach Anspruch 8, wobei sich die erste Mittelachse (C1) in eine erste Richtung relativ zu einer oberen/unteren Richtung erstreckt und sich die zweite Mittelachse (C2) in eine zweite entgegengesetzte Richtung relativ zur oberen/unteren Richtung erstreckt.

11. Kit nach Anspruch 8, wobei zumindest ein blockierendes Stellschraubenloch (125) durch das hintere Ende (114) des Implantats, angrenzend an zumindest eines der Befestigungslöcher (124), ausgebildet ist, wobei das blockierende Stellschraubenloch (125) eine siebte Mittelachse mit einer Ebene aufweist, die parallel zu den Ebenen der ersten (C1) und zweiten (C2) Achse in Querrichtung ist, und der Einsetzwerkzeugkörper (172) einen Mitnehmerdurchgang mit einer achten Mittelachse bildet, die eine Ebene aufweist, die mit der Ebene der siebten Mittelachse in Querrichtung ausgerichtet und parallel zu dieser ist, wenn das Implantat (110) auf dem Einsetzwerkzeug (170) abgestützt wird.

12. Kit nach Anspruch 11, das ferner eine blockierende Stellschraube (160) aufweist, die im blockierenden Stellschraubenloch (125) positioniert ist und zwischen einer nicht blockierenden Position und einer blockierenden Position durch einen im Mitnehmerdurchgang positionierten Mitnehmer beweglich ist.

13. Kit nach Anspruch 8, wobei zumindest ein Einsetzwerkzeug-Aufnahmeloch (125) durch das hintere Ende (114) ausgebildet ist und eine neunte Mittelachse mit einer Ebene aufweist, die parallel zu den Ebenen der ersten (C1) und zweiten (C2) Achse in Querrichtung ist, und sich ein Befestigungselement von der Fläche (172) des Einsatzwerkzeugkörpers (170) erstreckt und eingerichtet ist, im Einsetzwerkzeug-Aufnahmeloch (125) aufgenommen zu werden.

14. Kit nach Anspruch 8, das ferner ein Befestigungselement (140, 150) aufweist, das in jedem Befestigungsloch (124, 126) positioniert ist, wobei jedes der Befestigungselemente (140, 150) einen Schaft (142, 152) umfasst und die Schäfte (142, 152) in Ebenen liegen, die in Querrichtung parallel zueinander sind.

## Revendications

1. Implant intervertébral (110) destiné à être implanté dans un espace intervertébral entre des vertèbres, l'implant comprenant :
- un corps (111) ayant une extrémité avant (112), une extrémité arrière (114) et une paire de première et seconde parois latérales espacées (116, 118) s'étendant entre les extrémités avant (112) et arrière (114), les extrémités avant (112) et arrière (114) s'étendant dans une direction transversale et un axe central (CA) du corps (111) s'étendant de l'extrémité arrière (114) à l'extrémité avant (112),
- dans lequel l'extrémité arrière (114) définit un premier trou de fixation (124) ayant un premier axe central (C1) et un deuxième trou de fixation (126) ayant un deuxième axe central (C2), les premier (C1) et deuxième (C2) axes centraux se trouvant sur des plans respectifs s'étendant parallèlement l'un à l'autre et à un angle aigu par rapport à un plan sagittal (P2) de l'axe central du corps (CA) dans la direction transversale,
- dans lequel l'extrémité arrière (114) comprend un trou de réception d'outil d'insertion fileté (128) sur une partie adjacente à la seconde paroi latérale (118) et l'extrémité arrière (114) comprend en outre un trou borgne (127) avec une fente (129) sur une partie adjacente à la première paroi latérale (116), le trou de réception d'outil d'insertion fileté (128), le trou borgne (127) et la fente (129) étant configurés pour recevoir un outil d'insertion (170).

2. Implant intervertébral selon la revendication 1, dans lequel l'extrémité arrière (114) définit un troisième trou de fixation (124) ayant un troisième axe central (C3) situé sur un plan parallèle aux premier (C1) et deuxième axes (C2) dans la direction transversale.

3. Implant intervertébral selon la revendication 1, dans lequel le premier axe central (C1) s'étend dans une première direction par rapport à une direction supérieure/inférieure et le deuxième axe central (C2) s'étend dans une seconde direction opposée par rapport à la direction supérieure/inférieure.

4. Implant intervertébral selon la revendication 1, dans lequel au moins un trou de vis de blocage (125) est défini par l'extrémité arrière (114) adjacente à au moins l'un des trous de fixation (124), le trou de vis de blocage (125) ayant un quatrième axe central (C4) situé sur un plan parallèle aux premier (C1) et deuxième (C2) axes dans la direction transversale.

5. Implant intervertébral selon la revendication 4, comprenant en outre une vis de blocage (160) positionnée dans le trou de vis de blocage (125) et pouvant être déplacée entre une position de non-blocage et une position de blocage.

6. Implant intervertébral selon la revendication 1, dans lequel le trou de réception de l'outil d'insertion (128) a un cinquième axe central (C5) situé sur un plan parallèle aux premier (C1) et deuxième (C2) axes dans la direction transversale.

7. Implant intervertébral selon la revendication 1, comprenant en outre une fixation (140, 150) positionnée dans chaque trou de fixation (124, 126), chacune des fixations (140, 150) comprenant une tige (142, 152) et les tiges (142, 152) reposant sur des plans parallèles les uns aux autres dans la direction transversale.

8. Kit d'outil d'insertion (170) et d'implant intervertébral (110), le kit comprenant :
- un implant comprenant : un corps d'implant (111) ayant une extrémité avant (112), une extrémité arrière (114) et une paire de première et seconde parois latérales espacées (116, 118) s'étendant entre les extrémités avant (112) et arrière (114), les extrémités avant (112) et arrière (114) s'étendant dans une direction transversale et un axe central (CA) du corps d'implant (111) s'étendant de l'extrémité arrière (114) à l'extrémité avant (112), dans lequel l'extrémité arrière (114) définit un premier trou de fixation (124) ayant un premier axe central (C1) et un second trou de fixation (126) ayant un deuxième axe central (C2), les premier (C1) et deuxième (C2) axes centraux ayant des plans s'étendant de manière parallèle l'un à l'autre à un angle aigu par rapport à un plan de l'axe central (CA) du corps d'implant (111) dans la direction transversale, dans lequel l'extrémité arrière (114) comprend un trou de réception d'outil d'insertion fileté (128) sur une partie adjacente à la première paroi latérale (116) et l'extrémité arrière (114) comprend en outre un trou borgne (127) avec une fente (129) sur une partie adjacente à la seconde paroi latérale (118), dans lequel le trou de réception d'outil d'insertion fileté (128), le trou borgne (127), et la fente (129) sont configurés pour recevoir un outil d'insertion (170) ; et
- un outil d'insertion (170) comprenant : un corps d'outil (172) s'étendant d'une extrémité proximale (171) à une extrémité distale (173), l'extrémité distale (173) définissant une face (175) et le corps d'outil (172) définissant au moins deux passages de fixation avec des troisième (C4) et quatrième (C5) axes centraux respectifs,
- dans lequel l'outil d'insertion (170) est configuré pour soutenir l'implant (110) de sorte que l'extrémité arrière de l'implant (114) s'étende le long de la face (175) et que les plans des troisième (C4) et quatrième (C5) axes centraux s'alignent sur les plans des premier (C1) et deuxième (C2) axes centraux, respectivement, et soient parallèles à ceux-ci, dans la direction transversale.

9. Kit selon la revendication 8, dans lequel l'extrémité arrière de l'implant (114) définit un troisième trou de fixation (124) ayant un cinquième axe central (C3) avec un plan qui est parallèle aux plans des premier (C1) et deuxième (C2) axes dans la direction transversale et le corps de l'outil d'insertion (172) définit un troisième passage de fixation ayant un sixième axe central ayant un plan qui s'aligne avec le plan du cinquième axe central (C3), et est parallèle à celui-ci, dans la direction transversale lorsque l'implant (110) est supporté sur l'outil d'insertion.

10. Kit selon la revendication 8, dans lequel le premier axe central (C1) s'étend dans une première direction par rapport à une direction supérieure/inférieure et le deuxième axe central (C2) s'étend dans une seconde direction opposée par rapport à la direction supérieure/inférieure.

11. Kit selon la revendication 8, dans lequel au moins un trou de vis de blocage (125) est défini par l'extrémité arrière de l'implant (114) adjacente à au moins l'un des trous de fixation (124), le trou de vis de blocage (125) ayant un septième axe central dont le plan est parallèle aux plans des premier (C1) et deuxième (C2) axes dans la direction transversale et le corps de l'outil d'insertion (172) définit un passage d'entraînement ayant un huitième axe central dont le plan est aligné et parallèle au plan du septième axe central dans la direction transversale lorsque l'implant (110) est supporté par l'outil d'insertion (170).

12. Kit selon la revendication 11, comprenant en outre une vis de blocage (160) positionnée dans le trou de vis de blocage (125) et pouvant être déplacée entre une position de non-blocage et une position de blocage par un entraîneur positionné dans le passage de l'entraîneur.

13. Kit selon la revendication 8, dans lequel au moins un trou de réception d'outil d'insertion (125) est défini par l'extrémité arrière (114) et a un neuvième axe central ayant un plan qui est parallèle aux plans des premier (C1) et deuxième (C2) axes dans la direction transversale et un élément de montage s'étend de la face (172) du corps de l'outil d'insertion (170) et est configuré pour être reçu dans le trou de réception d'outil d'insertion (125).

14. Kit selon la revendication 8, comprenant en outre une fixation (140, 150) positionnée dans chaque trou de fixation (124, 126), chacune des fixations (140, 150) comprenant une tige (142, 152) et les tiges (142, 152) reposant sur des plans parallèles les uns aux autres dans la direction transversale.
